# EUROPEAN PATENT SPECIFICATION

(11) **EP 0 947 007 B1**
(45) Date of publication and mention of the grant of the patent: **05.12.2001**
(21) Application number: 97902011.2
(22) Date of filing: 14.01.1997
(51) Int. Cl.: H01M 2/02, H01M 2/10

(54) **STERILIZABLE BATTERY ENCLOSURE**
STERILISIERBARE BATTERIEUMKLEIDUNG
ENCEINTE DE PILE STERILISABLE

(30) Priority: 05.08.1996 US 693917
(43) Date of publication of application: 06.10.1999
(73) Proprietor: LINVATEC CORPORATION, Largo, Florida 33773 (US)
(72) Inventor: KLINZING, William, P., Saint Paul, MN 55133-3427 (US); DRAKE, Gerald, E., Saint Paul, MN 55133-3427 (US)
(74) Representative: VOSSIUS & PARTNER
(86) International application number: US9700525
(87) International publication number: WO9806144

(56) References cited:
- EP-A- 0 238 204
- EP-A- 0 255 631
- DE-A- 3 608 815
- US-A- 4 091 880
- US-A- 4 288 733

## Description

### Field of the Invention

The present invention relates to battery powered surgical tools, and in particular to a sterilizable battery enclosure for a battery installed on a surgical drive assembly and a method for isolating a nonsterile battery from a sterile field.

### Background of the Invention

Rechargeable battery-powered surgical drive assemblies are well known in the art. They are commonly used for orthopedic procedures such as drilling, screwing, reaming, wire driving, pinning, and sawing. Typically, such battery-powered surgical drive assemblies are generally pistol-shaped, having a drive portion, to which attachments are fitted, and a pistol-grip handle. Typically, the battery or battery pack ("battery") is detachably mounted ("installed") on the butt of the pistol grip. Examples of such battery-powered surgical drive assemblies are the "Orthopower 90" devices available from Stryker® (Kalamazoo, MI) and the "Maxi-IIe" device available from Minnesota Mining and Manufacturing ("3M") (St. Paul, MN).

Surgical drive assemblies, exclusive of the battery, are typically designed and constructed to withstand repeated sterilization by autoclaving. Autoclaving of rechargeable batteries, however, significantly shortens battery life. Because the batteries are expensive, and their disposal involves hazardous waste problems, there is a need to lengthen the useful life of batteries used in surgical drive assemblies.

### Summary of the Invention

The sterilizable battery enclosure and method of the present invention are characterized by the features of the claims.

In general, the invention features a sterilizable battery enclosure that isolates a nonsterile battery on a surgical drive assembly from the surrounding sterile field. The invention also features a method for isolating a nonsterile battery on a surgical drive assembly from the surrounding sterile field, when the drive assembly is used in surgery. The invention eliminates the need to subject the battery to sterilization procedures, such as autoclaving, which can severely shorten the useful life of a rechargeable battery.

The sterilizable battery enclosure includes two basic components. The first basic component is a battery sleeve having a proximal portion with an open proximal end, and a distal portion with a closed distal end. The battery sleeve has a size and shape that substantially conforms to the outside dimensions of the battery.

The second basic component is a bridging means that tightly couples the battery sleeve and the surgical drive assembly. The bridging means can be a flexible, resilient boot, or an adapter.

The boot has a proximal portion constituting a boot neck capable of forming a water-tight seal around the portion of the drive assembly adjacent to the battery. The boot has a distal portion constituting a boot skirt capable of being rolled up when the boot neck is in place on the drive assembly.

When: (a) the boot neck is in place on the drive assembly, (b) the boot skirt is rolled up, (c) the battery is installed, and (d) the sleeve is on the battery, the boot skirt unrolls over the proximal portion of the battery sleeve, forming a tight overlap. This prevents any portion of the nonsterile battery from being exposed to the surrounding environment.

Some embodiments of the present invention include an adapter for use on an irregularly-shaped pistol-grip. The adapter attaches to the surgical drive assembly in place of the battery, and then the battery attaches to the adapter.

The battery sleeve can be made of a nonwoven fabric, which is preferably impermeable to aqueous fluids. When the battery sleeve is made of fabric, it typically includes folds and seams, which preferably are substantially impermeable to aqueous fluids.
Alternatively, the battery sleeve can be rigid.
Preferably, a rigid battery sleeve is made of thermoform plastic, injection-molded plastic, or metal.

The sterilizable battery enclosure is typically used on a battery that mounts on the butt of a pistol-grip. The boot can be made of any elastomeric polymer or thermoplastic rubber. Preferably, the boot is made of a thermoplastic resin. More preferably, the thermoplastic resin is polyvinylchloride. Typically, the thickness of the boot is from about 0.25 mm (0.010 inch) to about 2.54 mm (0.100 inch). When the boot is made of polyvinylchloride, its thickness is preferably from about 0.51 mm (0.020 inch) to about 1.27 mm (0.050 inch).

The adapter has: (a) a proximal portion that mimics installation fittings on the battery, (b) a distal portion that mimics battery installation fittings on the surgical drive assembly, and (c) electrical terminals on the proximal and distal portions of the adapter. Typically, the adapter is installed on the butt of a pistol-grip. Preferably, the adapter has a circumferential lip under which the open end of a rigid battery sleeve fits snugly. Preferably, the rigid battery sleeve snaps onto the adapter.

A first method for isolating a nonsterile battery from a sterile field, during use of the battery to power a sterile surgical drive assembly includes the steps of: (a) placing on a portion of the surgical drive assembly adjacent to the battery mounting position a flexible, resilient boot, with a proximal portion constituting a boot neck capable of forming a water-tight seal around the portion of the drive assembly on which the battery is installed, and a distal portion constituting a boot skirt capable of being rolled up when the neck is sealed around the portion of the drive assembly; (b) installing a nonsterile battery on a sterile surgical drive assembly having a sterile boot in place; (c) placing on the battery a sterile battery sleeve having a proximal portion with an open proximal end, a closed distal end, and a size and shape substantially conforming to the outside dimensions of the battery; (d) unrolling the boot skirt onto the proximal portion of the battery sleeve, forming a tight overlap.

A second method for isolating a nonsterile battery from a sterile field, during use of the battery to power a sterile surgical drive assembly includes the steps of: (a) installing on the surgical drive assembly an adapter having: (1) a proximal portion that mimics installation fittings on the battery, (2) a distal portion that mimics battery installation fittings on the surgical drive assembly, and (3) electrical terminals on the proximal and distal portions of the adapter; (b) installing a nonsterile battery on the installed adapter, when the installed adapter and the surgical drive assembly are sterile; (c) placing over the battery a sterile, rigid battery sleeve having (1) a size and shape substantially conforming to the outside dimensions of the battery, (2) a proximal portion with an open proximal end, and (3) a closed distal end, so that the open proximal end of the rigid battery sleeve fits snugly against the adapter.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, the preferred methods and materials are described below. All publications, patent applications, patents, and other references mentioned herein are incorporated by reference in their entirety. In case of conflict, the present document, including definitions, will control. Unless otherwise indicated, materials, methods, and examples described herein are illustrative only and not intended to be limiting. Various features and advantages of the invention will be apparent from the following detailed description and from the claims.

### Brief Description of the Drawings

Fig. 1 is a plan view of a Stryker-type surgical drive assembly with boot in rolled-up position, and a detached battery with battery sleeve in place.

Fig. 2 is a plan view of a Stryker-type surgical drive assembly with battery installed, battery sleeve in place, and boot unrolled over the proximal (upper) portion of the battery sleeve.

Fig. 3 is a plan view of 3M-type drive assembly with boot in rolled-up position, detached battery, and battery sleeve.

Fig. 4 is a plan view of 3M-type drive assembly with boot in rolled-up position, and installed battery with battery sleeve in place.

Fig. 5 is a plan view of 3M-type drive assembly with installed battery and boot unrolled over proximal (upper) portion of battery sleeve.

Fig. 6 is a plan view of a 3M-type drive assembly, an adapter that fits on the pistol grip, and a battery that fits on the adapter. The adapter facilitates the use of a battery enclosure on an irregularly-shaped pistol-grip.

Fig. 7 is a plan view of a 3M-type drive assembly with an adapter and battery attached. This figure also shows a rigid battery sleeve that snaps onto the adapter.

### Detailed Description

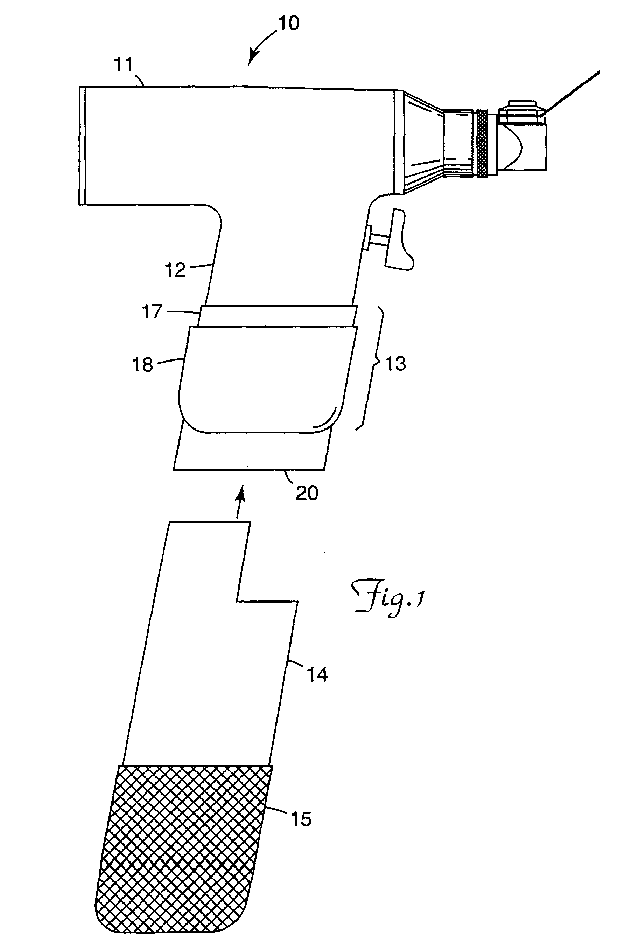

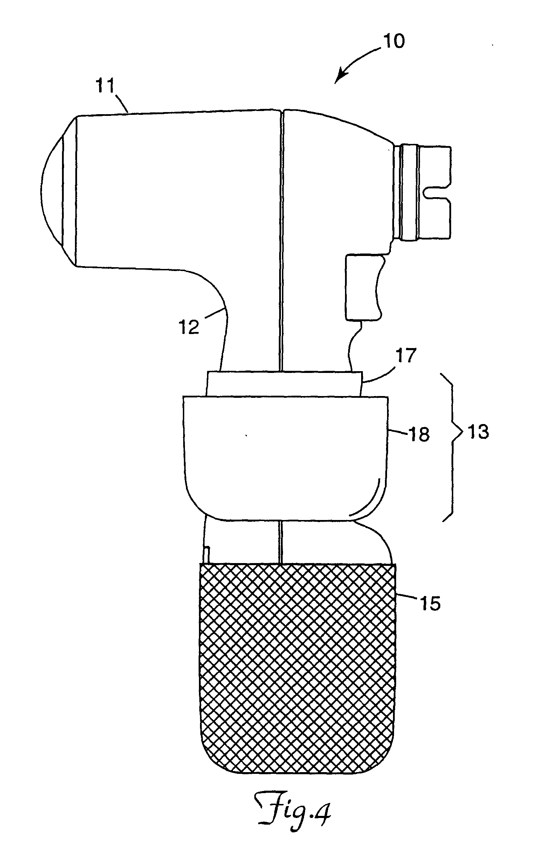

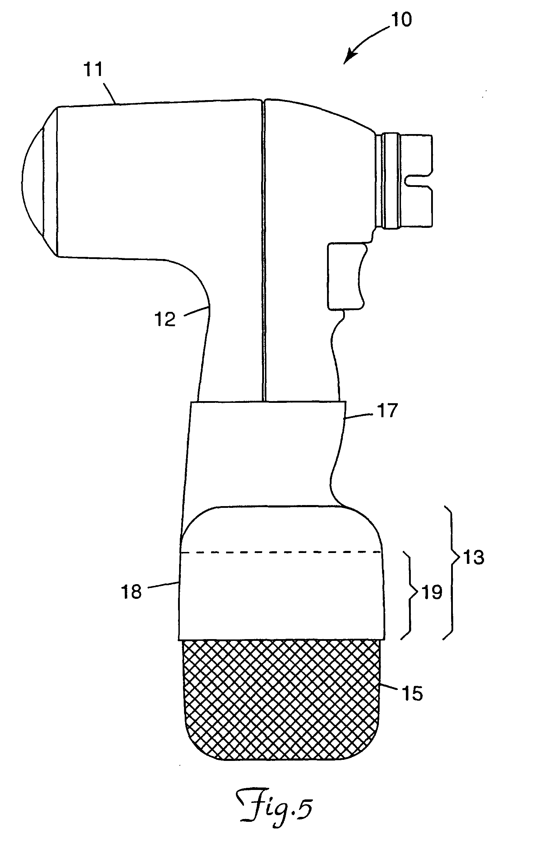

Referring now to Figures 1-7, the invention provides a sterilizable battery enclosure that isolates a nonsterile battery **14** on a surgical drive assembly **10** from the surrounding sterile field, during surgery. By removing the need to sterilize rechargeable batteries used on surgical drive assemblies, the invention significantly extends battery life. The sterilizable battery enclosure does not interfere with normal use of the surgical drive assembly, because the enclosure is lightweight and non-bulky.

The sterilizable battery enclosure includes: (1) a battery sleeve **15, 35**; and (2) a flexible, resilient boot **13**, or an adapter **30**. When a boot-type battery enclosure is in use, the boot **13** forms a tight overlap **16** completely around the open end **21** of the battery sleeve. When an adapter-type battery enclosure is in use, the adapter **30** connects securely to the battery sleeve **35**, preferably, without a boot.

Various agents are used to sterilize surgical instruments, including heat (autoclaving), gamma radiation, gas plasma, and chemical vapor, e.g., ethylene oxide, hydrogen peroxide, or peracetic acid. The flexible, resilient boot **13** and the battery sleeve **15** of the present invention must each be sterilizable by at least one regulatory agency-approved method of sterilization. If an adapter **30** is used to facilitate the use of the battery enclosure on an irregularly-shaped pistol-grip, the adapter **30** also must be sterilizable.

### The Battery Sleeve

The battery sleeve **15** forms the distal (typically the lower) portion of the battery enclosure. The sleeve's essential function is to serve as a barrier between the nonsterile battery **14** and the surrounding environment. The battery sleeve **15** must be made of a sterilizable material. Preferably, the sterilizable material is substantially impermeable to bacteria, viruses, and aqueous liquids, e.g., blood. As used herein, "substantially impermeable" means that upon contact between the barrier material and an aqueous liquid, measurable permeation does not occur in less than about one hour.

In one preferred embodiment of the invention, the battery sleeve **15** is made of a sheet of nonwoven fabric folded and seamed to fit the battery **14.** The preferred fabric for use in such an embodiment is surgical drape fabric. A particularly preferred fabric is the surgical drape fabric commercially available as "3M Biocade Fabric" (Minnesota Mining and Manufacturing, St. Paul, MN). Alternatively, a folded and seamed battery sleeve can be made of a thin sheet of plastic.

When the battery sleeve is made from folded and seamed fabric or thin plastic, the seams are preferably impermeable to microparticulates and aqueous liquids. Impermeable seams can be made by conventional techniques such as gluing or ultrasonic welding.

In an alternative embodiment of the invention, the battery sleeve is rigid. When the battery sleeve is rigid, it is preferably a single, seamless piece of smooth material. Preferably, corners and edges on a rigid battery sleeve are smoothly rounded, if the rigid battery sleeve is used with a boot. This reduces the likelihood of gapping, when the boot skirt is unrolled onto the battery sleeve to form a tight overlap. Preferably, the overlap between the boot skirt and the rigid battery sleeve is water-tight. A preferred rigid battery sleeve is made of thermoform plastic or injection-molded plastic. A rigid battery sleeve can also be made of metal, e.g., surgical stainless steel.

Fabrics and plastics offer the advantage of light weight. In addition, fabric or plastic battery sleeves are suitable for sale as sterile, single-use, disposable items. Stainless steel battery sleeves are suitable for autoclaving and essentially unlimited reuse.

The battery sleeve must substantially conform to the outside dimensions of the battery on which it is used. Such conformity avoids bulkiness, which could impede a surgeon's movements or cause unnecessary physical contact between the battery sleeve and its surroundings during use. As used herein, "substantially conforming" to the outside dimensions of the battery means that the gap between any external surface on the battery, and the inside surface of the sleeve, does not exceed 6.35 mm (one quarter inch). Preferably, the gap does not exceed 3.175 mm (one eighth inch).

Any gaps between the upper portion of a folded and seamed battery sleeve and the upper portion of the battery are eliminated as the sleeve is compressed against the sides of the battery by the tight-fitting boot skirt. Any gaps between the proximal portion of a rigid battery sleeve and the proximal portion of the battery must be isolated from the surrounding sterile field. Such isolation can be achieved, for example, by a tight fit between a boot skirt and the outside surface of the proximal portion of a rigid battery sleeve. Alternatively, such isolation can be achieved by a tight fit between an adapter and the open end of a rigid battery sleeve.

### The Boot

The boot **13** is a generally tubular piece with a proximal portion **17**, i.e., the "boot neck," and a distal portion **18**, i.e., the "boot skirt." In essence, the sterile boot **13** serves as a bridge, tightly coupling the sterile battery sleeve **15** and the sterile surgical drive assembly **10**. The boot neck **17** forms a water-tight seal around a portion of the surgical drive assembly **10** adjacent to the battery. The boot skirt **18** forms a tight overlap **16** completely around the open end of the battery sleeve **15**. The boot **13** inherently forms the necessary water-tight seal and tight overlap **16**, by virtue of its geometry and material composition, without the use of tape, a clamp, or any type of fastener.

A battery-powered surgical drive assembly **10** is typically pistol-shaped, with a chuck at the front end of the barrel, and the battery **14** installed on the butt **20** of the pistol-grip **12**. Thus, with a typical surgical drive assembly, the boot neck **17** forms a seal around a pistol-grip **12**. When the surgical drive assembly **10** has the battery mounted elsewhere, e.g., at the rear end of the barrel, the boot forms a seal around the portion of the drive assembly to which the battery is mounted. As used herein, "battery installed on the butt" of a pistol-grip includes an arrangement in which a portion of the battery is inside a hollow pistol grip.

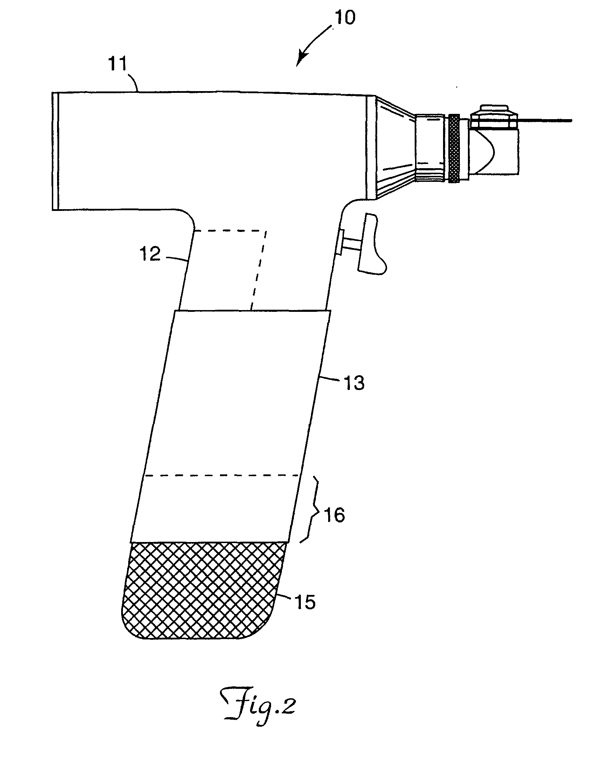

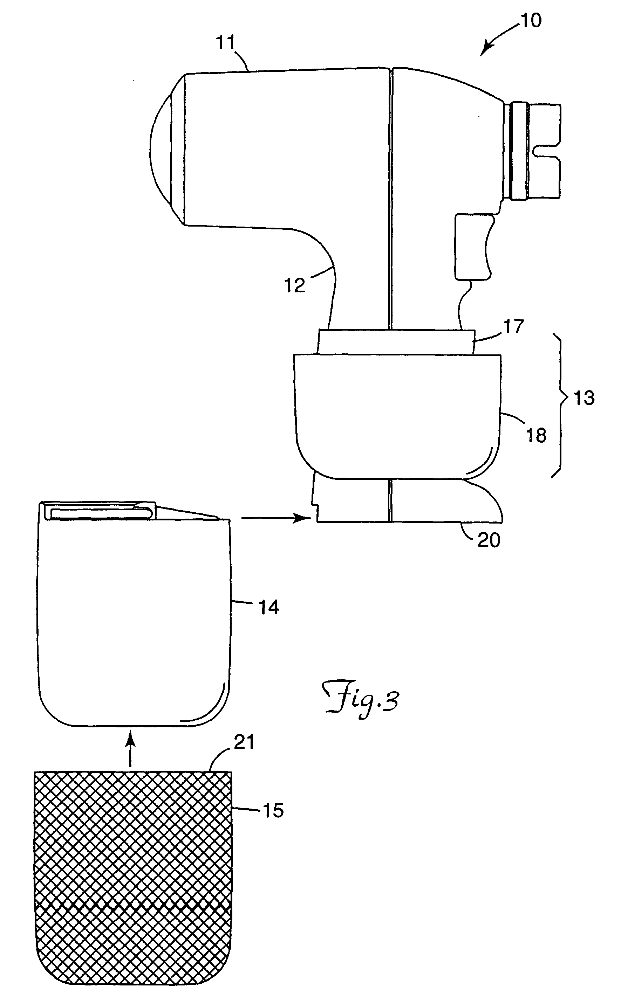

A well-known type of surgical drive assembly, sold by Stryker, has a hollow pistol-grip (Figs. 1 and 2). The upper portion of an elongate battery slides vertically into the pistol-grip, with the lower portion of the battery protruding below the butt **20** of the pistol-grip. Another well-known type of surgical drive assembly, sold by Minnesota Mining and Manufacturing Co., has a pistol-grip **12** that is not hollow (Figs. 3-5). The battery **14** slides horizontally onto a fitting on the butt **20** of the pistol-grip.

The boot **13** is flexible, resilient, and impermeable to aqueous liquids. Preferably, it has a small amount of elasticity, which facilitates installation and tight sealing at the top and bottom. Preferably, the boot **13** does not easily slide on a hard, smooth surface such as metal or hard plastic, i.e., it exhibits substantial friction when in contact with a hard, smooth surface. Substantial friction is desirable for keeping the boot **13** securely in place during use.

The boot **13** can be made of any sterilizable, flexible, resilient material capable of providing a water-tight seal at the boot neck **17**, and a tight overlap at the boot skirt **18**. Preferred boot materials are elastomeric polymers. More preferred boot materials are thermoplastic resins. The most preferred boot material is polyvinylchloride. A one-piece boot suitable for use in the present invention can be made from polyvinylchloride by conventional dip molding techniques. The advantages of polyvinylchloride include the ability to withstand repeated sterilization by autoclaving, or other methods, without significant deterioration in performance.

When the boot is made of dip-molded polyvinylchloride, it is preferable to use a plastisol compounded from a polyvinylchloride homopolymer dispersion resin with a particle size between about 0.2 and 15 micrometers. This material is dispersed in a plasticizer, such as a phthalic acid ester. Esters of alkyl alcohols such as dioctyl phthalate can be used. Alternatively, esters of aromatic alcohols such as butyl benzyl phthalate can be used, especially when a thicker boot is desirable.

The ratio of ingredients will vary depending on the plasticizers chosen, and the desired hardness of the finished boot. A hardness between about 5 and about 95 Shore A is preferred. More preferably, the hardness is between about 40 and about 70 Shore A. For many ingredient selections, a ratio of approximately 70 parts (by weight) of plasticizer per 100 parts (by weight) of dispersion resin will yield a boot having the desired properties.

A heat stabilizer is required in the dipping compound, and a pigment optionally can be added. To obtain a long wearing part, it is important that the boot be cured fully after dipping of the mold in the described plastisol. A curing temperature of between about 177°C and about 191°C (about 350° F and about 375° F), for approximately six minutes, is suitable.

The inside dimensions of the boot **13** are determined primarily by: (1) the outside dimensions of the portion of the surgical drive assembly to which the boot attaches, e.g., the handle **12**; and (2) the outside dimensions of the battery sleeve **15,** when the sleeve is on the battery **14.** The boot's flexibility, resiliency, and elasticity can also affect its dimensions. An elastic boot **13** can have relatively small dimensions in its relaxed state, and accommodate the surgical drive assembly and battery-containing sleeve **15** by stretching.

Optimal boot thickness will depend primarily on the material from which the boot **13** is formed. In general, for a given material, the minimum thickness that will provide an acceptable level of strength and durability is preferred. Excessive thickness increases bulkiness and decreases flexibility. Excessive thickness also makes it more difficult to roll the boot skirt **18** up and down. Typically, boot thickness is from 0.25 mm (0.010 inch) to 2.5 mm (0.100 inch). When the boot **13** is made of polyvinylchloride, its thickness is preferably from 0.51 mm (0.020 inch) to 1.27 mm (0.050 inch).

The boot skirt **18** rolls up to facilitate its placement over the proximal portion of the battery sleeve, by unrolling. Unrolling the boot skirt over the proximal portion of the battery sleeve makes it possible to use a boot material and a battery sleeve material that do not slide on each other when in tight contact. Preferably, the boot is placed on the surgical drive assembly **10** prior to sterilization, and then the drive assembly-boot combination is sterilized as a unit. The boot **13** can be left in place on the drive assembly **10** for repeated cycles of use and sterilization.

### The Adapter

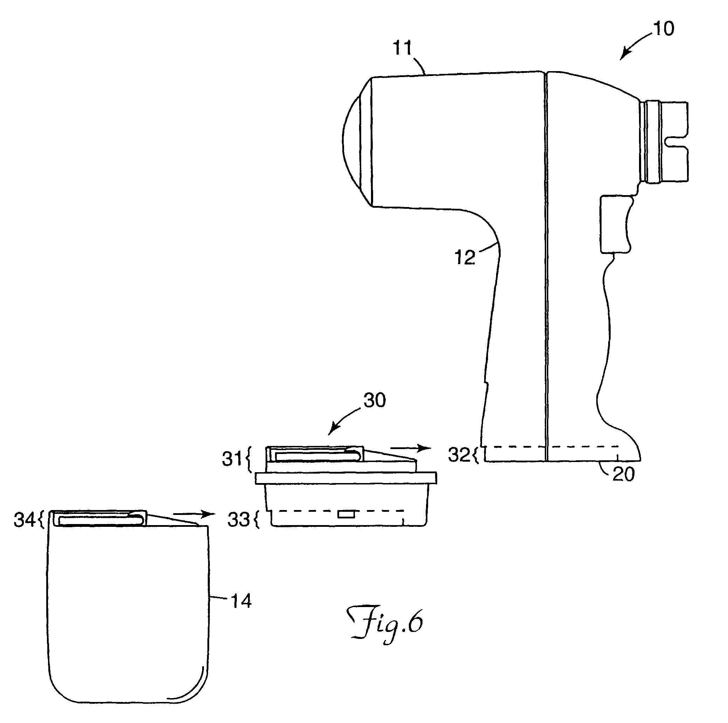

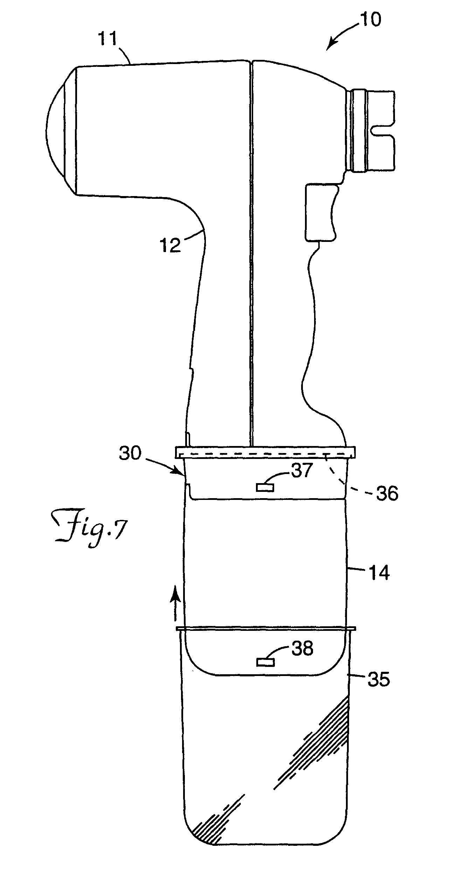

When the surgical drive assembly has a pistol-grip with a regular shape, such as that shown in Figs. 1 and 2, the preferred bridging means is a flexible boot **13**. When the surgical drive assembly has a pistol-grip with an irregular shape, such as that shown in Figs. 3-7, the preferred bridging means is an adapter **30** (Figs. 6 and 7). In a preferred embodiment of the invention, the battery sleeve **35** is rigid and fastens directly onto the adapter **30**, with a boot being unnecessary. Optionally, a boot can be used to overlap the junction between the adapter **30** and the battery **14**.

The adapter **30** has an upper portion **31**, which mimics the installation fittings **34** on the battery, and a lower portion **33**, which mimics the battery installation fittings **32** on the pistol grip. Thus, the upper portion **31** of the adapter **30** attaches to the pistol grip **12** in place of the battery **14**, and the battery **14** attaches to the adapter **30.** The adapter **30** must have suitable electrical terminals on its upper and lower portions **31**, **33**, so that the battery is operably connected to the surgical drive assembly.

Preferably, the adapter has a circumferential lip **36** under which the open end of a rigid battery sleeve **35** fits snugly. The lip **36** advantageously prevents the flow of liquids between the pistol grip **12** and the inside of the battery sleeve **35**. In preferred embodiments, a rigid battery sleeve **35** snaps securely onto the adapter **30**. Such snapping can be by means of a protrusion **37** on the adapter which fits into an indentation **38** on the rigid battery sleeve **35**. Preferably, the size and geometry of the adapter **30** generally conform to the size and geometry of the lower portion of the pistol grip **12**, with the installed adapter **30** adding minimally to the length of the pistol grip **12**.

The adapter **30** can be made of any hard, smooth, sterilizable material. Preferably, the body of the adapter **30** is made of a material that is lightweight and is an electrical insulator. A preferred material for the body of the adapter is RADEL R-5100 (Amoco). If the body of the adapter is not made of an electrically-insulating material, a means for insulating the electrical components of the adapter from the body of the adapter must be included. The design of such insulating means is within ordinary skill in the art.

Other embodiments are within the following claims.

## Claims

1. A sterilizable battery enclosure for a battery installed on a surgical drive assembly, comprising
(a) a battery sleeve with a proximal portion having an open proximal end, and a closed distal end, said sleeve having a size and shape substantially conforming to the outside dimensions of said battery;
(b) a bridging means that tightly couples the battery sleeve and the surgical drive assembly, so as to prevent any portion of said battery from being exposed to the surrounding environment, said bridging means selected from the group consisting of a flexible, resilient boot, and an adapter.

2. The sterilizable battery enclosure of claim 1, wherein said bridging means is a flexible, resilient boot with a proximal portion constituting a boot neck capable of forming a water-tight seal around the portion of said drive assembly on which said battery is installed, and a distal portion constituting a boot skirt- capable of being rolled up when said neck is sealed around said portion of said drive assembly;
wherein, when: (a) said boot neck is installed on said drive assembly, (b) said boot skirt is rolled up, (c) said battery is installed on said surgical drive assembly, and (d) said sleeve is installed on said battery; said boot skirt unrolls onto said proximal portion of said sleeve, forming a tight overlap.

3. The sterilizable battery enclosure of claim 2, wherein the portion of said drive assembly on which said battery is installed is the butt of a pistol-grip.

4. The sterilizable battery enclosure of claim 1, wherein said bridging means is an adapter having: (a) a proximal portion that mimics installation fittings on said battery, (b) a distal portion that mimics battery installation fittings on said surgical drive assembly, and (c) electrical terminals on said proximal and distal portions.

5. The sterilizable battery enclosure of claim 4, wherein said battery is installed on the butt of a pistol-grip.

6. The sterilizable battery enclosure of claim 4, wherein said adapter has a circumferential lip under which the open end of a rigid battery sleeve fits snugly.

7. The sterilizable battery enclosure of claim 6, wherein said rigid battery sleeve snaps onto said adapter.

8. A method for isolating a nonsterile battery from a sterile field, during use of said nonsterile battery to power a sterile surgical drive assembly, comprising the steps of:
(a) placing on a portion of said surgical drive assembly adjacent to the battery mounting position a flexible, resilient boot, with a proximal portion constituting a boot neck capable of forming a water-tight seal around the portion of said drive assembly on which said battery is installed, and a distal portion constituting a boot skirt capable of being rolled up when said neck is sealed around said portion of said drive assembly;
(b) installing a nonsterile battery on said surgical drive assembly with boot in place, said surgical drive assembly and boot being sterile;
(c) placing on said battery a sterile battery sleeve with a proximal portion having an open proximal end, and a closed distal end, said sleeve having a size and shape substantially conforming to the outside dimensions of said battery;
(d) unrolling said boot skirt onto said proximal portion of said battery sleeve, forming a tight overlap;
thereby isolating said nonsterile battery from said sterile field.

9. The method of claim 8, wherein said battery mounting position is the butt of a pistol-grip.

10. The battery enclosure or method of claim 2 or 8, wherein said flexible resilient boot is made of a thermoplastic resin.

11. The battery enclosure or method of claim 10, wherein said thermoplastic resin is polyvinylchloride.

12. The battery enclosure or method of any of claims 2 to 11, wherein said flexible, resilient boot has a thickness from about 0.020 inch to about 0.050 inch.

13. The sterilizable battery enclosure or method of any of claims 2 to 12, wherein said battery sleeve is made of a material selected from the group consisting of nonwoven fabric, woven fabric, and flexible plastic, said material being substantially impermeable to aqueous fluids.

14. The battery enclosure or method of claim 13, wherein said nonwoven fabric is "3M Biocade Fabric".

15. The battery enclosure or method of claim 13, wherein said battery sleeve includes folds and seams.

16. The battery enclosure or method of claim 15, wherein said seams are substantially impermeable to aqueous fluids.

17. The battery enclosure or method of claim 1 or 8, wherein said battery sleeve is rigid.

18. The battery enclosure or method of claim 17, wherein said battery sleeve is plastic, preferably thermoform or injection-molded plastic.

19. The battery enclosure or method of claim 17, wherein said battery sleeve is metal.

20. A method for isolating a nonsterile battery from a sterile field, during use of said nonsterile battery to power a sterile surgical drive assembly, comprising the steps of:
(a) installing on said surgical drive assembly an adapter having: (1) a proximal portion that mimics installation fittings on said battery, (2) a distal portion that mimics battery installation fittings on said surgical drive assembly, and (3) electrical terminals on said proximal and distal portions;
(b) installing a nonsterile battery on the installed adapter, when said installed adapter and said surgical drive assembly are sterile;
(c) placing over said battery a sterile, rigid battery sleeve with a proximal portion having an open proximal end, and a closed distal end, said sleeve having a size and shape substantially conforming to the outside dimensions of said battery, said open proximal end of said rigid battery sleeve fitting snugly against said adapter,
thereby isolating a nonsterile battery from a sterile field.

21. The method of claim 20,wherein said adapter has a circumferential lip under which said open end of said rigid battery sleeve fits snugly.

## Patentansprüche

1. Sterilisierbare Batterieumkleidung für eine an einer chirurgischen Antriebseinheit installierte Batterie, wobei die Batterieumkleidung aufweist:
(a) eine Batteriehülse mit einem proximalen Abschnitt, der ein offenes proximales Ende aufweist, und einem geschlossenen distalen Ende, wobei die Hülse eine Größe und eine Form aufweist, die weitgehend mit den Außenabmessungen der Batterie übereinstimmen;
(b) eine Überbrückungseinrichtung, welche die Batteriehülse und die chirurgische Antriebseinheit fest miteinander verbindet, um zu verhindern, daß irgendein Abschnitt der Batterie der Umgebung ausgesetzt wird, wobei die Überbrückungseinrichtung aus der Gruppe ausgewählt ist, die aus einer flexiblen, elastischen Haube und einem Adapter besteht.

2. Sterilisierbare Batterieumkleidung nach Anspruch 1, wobei die Überbrückungseinrichtung eine flexible, elastische Haube ist, mit einem proximalen Abschnitt, der einen Haubenhals bildet, der eine wasserdichte Abdichtung rund um den Abschnitt der Antriebseinheit bilden kann, an dem die Batterie installiert ist, und einem distalen Abschnitt, der eine Haubenschürze bildet, die aufgerollt werden kann, wenn der Hals rund um den Abschnitt der Antriebseinheit abgedichtet ist;
wobei, wenn (a) der Haubenhals an der Antriebseinheit installiert ist, (b) die Haubenschürze aufgerollt ist, (c) die Batterie an der chirurgischen Antriebseinheit installiert ist und (d) die Hülse auf der Batterie installiert ist, die Haubenschürze auf den proximalen Abschnitt der Hülse abgerollt wird und eine dichte Überdeckung bildet.

3. Sterilisierbare Batterieumkleidung nach Anspruch 2, wobei der Abschnitt der chirurgischen Antriebseinheit, an dem die Batterie installiert ist, das stumpfe Ende eines Pistolengriffs ist.

4. Sterilisierbare Batterieumkleidung nach Anspruch 1, wobei die Überbrückungseinrichtung ein Adapter ist, der aufweist: (a) einen proximalen Abschnitt, der Paßteile an der Batterie nachbildet, (b) einen distalen Abschnitt, der Batteriepaßteile an der chirurgischen Antriebseinheit nachbildet, und (c) elektrische Anschlüsse an dem proximalen und dem distalen Abschnitt.

5. Sterilisierbare Batterieumkleidung nach Anspruch 4, wobei die Batterie am stumpfen Ende eines Pistolengriffs installiert ist.

6. Sterilisierbare Batterieumkleidung nach Anspruch 4, wobei der Adapter eine Umfangslippe aufweist, unter der das offene Ende einer starren Batteriehülse genau passend anliegt.

7. Sterilisierbare Batterieumkleidung nach Anspruch 6, wobei die starre Batteriehülse an dem Adapter einrastet.

8. Verfahren zum Isolieren einer nichtsterilen Batterie von einem sterilen Bereich während der Verwendung der nichtsterilen Batterie zum Betrieb einer sterilen chirurgischen Antriebseinheit, mit den folgenden Schritten:
(a) Anbringen einer flexiblen, elastischen Haube an einem an die Batteriemontageposition angrenzenden Abschnitt der chirurgischen Antriebseinheit, wobei ein proximaler Abschnitt einen Haubenhals bildet, der eine wasserdichte Abdichtung rund um den Abschnitt der Antriebseinheit bilden kann, an dem die Batterie installiert wird, und mit einem distalen Abschnitt, der eine Haubenschürze bildet, die aufgerollt werden kann, wenn der Hals rund um den Abschnitt der Antriebseinheit abgedichtet wird;
(b) Installieren einer nichtsterilen Batterie an der chirurgischen Antriebseinheit mit aufgesetzter Haube, wobei die chirurgische Antriebseinheit und die Haube steril sind;
(c) Anbringen einer sterilen Batteriehülse, die einen proximalen Abschnitt mit einem offenen proximalen Ende und ein geschlossenes distales Ende aufweist, an der Batterie, wobei die Hülse eine Größe und eine Form aufweist, die weitgehend mit den Außenabmessungen der Batterie übereinstimmen;
(d) Abrollen der Haubenschürze auf den proximalen Abschnitt der Batteriehülse zum Ausbilden einer dichten Überdekkung;
wodurch die nichtsterile Batterie von dem sterilen Bereich isoliert wird.

9. Verfahren nach Anspruch 8, wobei die Batteriemontageposition das stumpfe Ende eines Pistolengriffs ist.

10. Batterieumkleidung oder Verfahren nach Anspruch 2 oder 8, wobei die flexible, elastische Haube aus einem thermoplastischen Harz besteht.

11. Batterieumkleidung oder Verfahren nach Anspruch 10, wobei das thermoplastische Harz Polyvinylchlorid ist.

12. Batterieumkleidung oder Verfahren nach einem der Ansprüche 2 bis 11, wobei die flexible, elastische Haube eine Dicke von etwa 0,508 mm (0,020 Zoll) bis etwa 1,27 mm (0,050 Zoll) aufweist.

13. Sterilisierbare Batterieumkleidung oder Verfahren nach einem der Ansprüche 2 bis 12, wobei die Batteriehülse aus einem Material besteht, das aus der Gruppe ausgewählt ist, die aus Vliesstoff, Gewebe und flexiblem Kunststoff besteht, wobei das Material im wesentlichen undurchlässig für wäßrige Fluide ist.

14. Batterieumkleidung oder Verfahren nach Anspruch 13, wobei der Vliesstoff "3M Biocade Fabric" ist.

15. Batterieumkleidung oder Verfahren nach Anspruch 13, wobei die Batteriehülse Falten und Nähte aufweist.

16. Batterieumkleidung oder Verfahren nach Anspruch 15, wobei die Nähte im wesentlichen undurchlässig für wäßrige Fluide sind.

17. Batterieumkleidung oder Verfahren nach Anspruch 1 oder 8, wobei die Batteriehülse starr ist.

18. Batterieumkleidung oder Verfahren nach Anspruch 17, wobei die Batteriehülse aus Kunststoff besteht, vorzugsweise aus warmformbarem oder spritzgegossenem Kunststoff.

19. Batterieumkleidung oder Verfahren nach Anspruch 17, wobei die Batteriehülse aus Metall besteht.

20. Verfahren zum Isolieren einer nichtsterilen Batterie von einem sterilen Bereich während der Verwendung der nichtsterilen Batterie zum Betrieb einer sterilen chirurgischen Antriebseinheit, mit den folgenden Schritten:
(a) Installieren eines Adapters an der chirurgischen Antriebseinheit, wobei der Adapter aufweist: (1) einen proximalen Abschnitt, der Paßteile an der Batterie nachbildet, (2) einen distalen Abschnitt, der Batteriepaßteile an der chirurgischen Antriebseinheit nachbildet, und (3) elektrische Anschlüsse an dem proximalen und dem distalen Abschnitt;
(b) Installieren einer nichtsterilen Batterie an dem installierten Adapter, sobald der installierte Adapter und die chirurgische Antriebseinheit steril sind;
(c) Anbringen einer sterilen, starren Batteriehülse mit einem proximalen Abschnitt, der ein offenes proximales Ende aufweist, und mit einem geschlossenen distalen Ende über der Batterie, wobei die Hülse eine Größe und eine Form aufweist, die weitgehend mit den Außenabmessungen der Batterie übereinstimmen, wobei das offene proximale Ende der starren Batteriehülse genau passend an dem Adapter anliegt,
wodurch eine nichtsterile Batterie von einem sterilen Bereich isoliert wird.

21. Verfahren nach Anspruch 20, wobei der Adapter eine Umfangslippe aufweist, unter der das offene Ende der starren Batteriehülse genau passend anliegt.

## Revendications

1. Une enceinte de pile stérilisable pour une pile installée sur un ensemble d'entraînement chirurgical, comprenant :
(a) une douille de pile, munie d'une partie proximale ayant une extrémité proximale ouverte, et une extrémité distale fermée, ladite douille ayant une taille et une forme se conformant sensiblement aux dimensions extérieures de ladite pile ;
(b) des moyens de pontage qui couplent de façon rigide la douille de pile et l'ensemble d'entraînement chirurgical, pour empêcher qu'une partie éventuelle de ladite pile soit exposée à l'environnement extérieur, lesdits moyens de pontage étant sélectionnés dans le groupe constitué d'un soufflet de protection élastique, flexible, et d'un adaptateur.

2. L'enceinte de pile stérilisable selon la revendication 1, dans laquelle lesdits moyens de pontage sont constitués d'un soufflet de protection élastique, flexible, ayant une partie proximale constituant un col de soufflet de protection susceptible de former un joint d'étanchéité à l'eau autour de la partie dudit ensemble d'entraînement sur lequel ladite pile est installée, et une partie distale, constituant une jupe de soufflet de protection susceptible d'être enroulée lorsque ledit col est fermé de façon étanchée autour de ladite partie dudit ensemble d'entraînement ; dans laquelle, lorsque : (a) ledit col de soufflet de protection est installé sur ledit ensemble d'entraînement, (b) ladite jupe de soufflet de protection est enroulée, (c) ladite pile est installée sur ledit ensemble d'entraînement chirurgical, et (d) ladite douille est installée sur ladite pile ; ladite jupe de soufflet de protection se déroule sur ladite partie proximale de ladite douille, en formant un élément de chevauchement étanche.

3. L'enceinte de pile stérilisable selon la revendication 2, dans laquelle la partie dudit ensemble d'entraînement sur laquelle ladite pile est installée est le culot d'une poignée de pistolet.

4. L'enceinte de pile stérilisable selon la revendication 1, dans laquelle les moyens de pontage sont constitués d'un adaptateur ayant : (a) une partie proximale qui reproduit les raccords d'installation sur ladite pile, (b) une partie distale qui reproduit les raccords d'installation de pile sur ledit ensemble d'entraînement chirurgical, et (c) des bornes électriques placées sur lesdites parties proximales et distales.

5. L'enceinte de pile stérilisable selon la revendication 4, dans lequel ladite pile est installée sur le culot d'une poignée de pistolet.

6. L'enceinte de pile stérilisable selon la revendication 4, dans lequel ledit adaptateur comporte une lèvre circonférentielle, sous laquelle l'extrémité ouverte de la douille rigide pour pile vient s'ajuster de façon affleurée.

7. L'enceinte de pile stérilisable selon la revendication 6, dans laquelle ladite douille rigide pour pile s'encliquette sur ledit adaptateur.

8. Un procédé d'isolation d'une pile non stérile envers un champ stérile, durant l'utilisation de ladite pile non stérile pour alimenter un ensemble d'entraînement chirurgical stérile, comprenant les étapes consistant à :
(a) placer, sur une partie dudit ensemble d'entraînement chirurgical, de façon adjacente à ladite position de montage de pile, un soufflet de protection élastique, flexible, ayant une partie proximale constituant un col de soufflet de protection, susceptible de former un joint d'étanchéité à l'eau autour de la partie dudit ensemble d'entraînement sur lequel ladite pile est installée, et une partie distale, constituant une jupe de soufflet de protection, susceptible d'être enroulée lorsque ledit col est scellé de façon étanche autour de ladite partie dudit ensemble d'entraînement ;
(b) installer une pile non stérile sur ledit ensemble d'entraînement chirurgical, le soufflet de protection étant en place, ledit ensemble d'entraînement chirurgical et ledit soufflet de protection étant stériles ;
(c) placer sur ladite pile une douille stérile de pile avec une partie proximale ayant une extrémité proximale ouverte et une extrémité distale fermée, ladite douille ayant une taille et une forme se conformant sensiblement aux dimensions extérieures de ladite pile ;
(d) dérouler ladite jupe de soufflet de protection sur ladite partie proximale de ladite douille de pile, en formant un chevauchement étanche ;
de manière à isoler ladite pile non stérile envers ledit champ stérile.

9. Le procédé selon la revendication 8, dans lequel ladite position de montage de pile est le culot d'une poignée de pistolet.

10. L'enceinte de pile ou le procédé selon la revendication 2 ou 8, dans lequel (laquelle) ledit soufflet de protection élastique flexible est réalisé en résine thermoplastique.

11. L'enceinte de pile ou le procédé selon la revendication 10, dans lequel (laquelle) ladite résine thermoplastique est du polychlorure de vinyle.

12. L'enceinte de pile ou le procédé selon l'une quelconque des revendications 2 à 11, dans lequel (laquelle) ledit soufflet de protection élastique flexible est d'une épaisseur d'environ 0,508 mm (0,020 pouce) à environ 1,27 mm (0,050 pouce).

13. L'enceinte pour pile stérilisable ou le procédé selon l'une quelconque des revendications 2 à 12, dans lequel (laquelle) ladite douille de pile est constituée d'un matériau sélectionné dans le groupe composé d'un textile non tissé, d'un textile tissé et d'une matière synthétique flexible, ledit matériau étant sensiblement imperméable aux fluides aqueux.

14. L'enceinte de pile ou le procédé selon la revendication 13, dans lequel (laquelle) ledit textile non tissé est du "3M Biocade Fabric".

15. L'enceinte de pile ou le procédé selon la revendication 13, dans lequel (laquelle) ladite enceinte de pile comprend des plis et des cordons de jointoiement.

16. L'enceinte de pile ou le procédé selon la revendication 15, dans lequel (laquelle) lesdits cordons de jointoiement sont sensiblement imperméables aux fluides aqueux.

17. L'enceinte de pile ou le procédé selon la revendication 1 ou 8, **caractérisé(e) en ce que** ladite douille de pile est rigide.

18. L'enceinte de pile ou le procédé selon la revendication 17, dans lequel (laquelle) ladite douille de pile et en matière synthétique, de préférence en matière synthétique thermoformable ou moulée par injection.

19. L'enceinte de pile ou le procédé selon la revendication 17, dans lequel (laquelle) ladite douille de pile est réalisée en métal.

20. Un procédé d'isolation d'une pile non stérile envers un champ stérile durant l'utilisation de ladite pile non stérile pour alimenter un ensemble d'entraînement chirurgical stérile, comprenant les étapes consistant à :
(a) installer sur ledit ensemble d'entraînement chirurgical un adaptateur comportant : (1) une partie proximale qui reproduit les raccords d'installation sur ladite pile, (2) une partie distale qui reproduit les raccords d'installation de pile sur ledit ensemble d'entraînement chirurgical, et (3) des bornes électriques sur lesdites parties proximales et distales ;
(b) installation d'une pile non stérile sur l'adaptateur installé, lorsque ledit adaptateur installé et ledit ensemble d'entraînement chirurgical sont stériles ;
(c) placement, sur ladite pile d'une douille de pile rigide stérile ayant une partie proximale, ayant une extrémité proximale ouverte et une extrémité distale fermée, ladite douille ayant une taille et une forme se conformant sensiblement aux dimensions extérieures de ladite pile, ladite extrémité proximale ouverte de ladite douille de pile rigide s'adaptant de façon affleurée contre ledit adaptateur,
de manière à isoler une pile non stérile envers un champ stérile.

21. Le procédé selon la revendication 20, dans lequel ledit adaptateur a une lèvre circonférentielle sous laquelle ladite extrémité ouverte de ladite douille de pile rigide s'ajuste de façon affleurée.
